# EUROPEAN PATENT APPLICATION

(11) **EP 1 382 334 A1**
(43) Date of publication of application: **21.01.2004**
(21) Application number: 02291752.0
(22) Date of filing: 11.07.2002
(51) Int. Cl.: A61K 31/4178, A61K 31/4184, A61K 31/41, A61K 31/549, A61K 38/08, A61P 9/00, A61P 25/00

(54) **Use of angiotensin II AT1-receptor blockers (ARB), alone or combined with thiazide or angiotensin II for the treatment of stroke**

(71) Applicant: Université de Picardie Jules Verne, 80000 Amiens (FR); Centre Hospitalo-Universitaire d' Amiens, 80080 Amiens (FR)
(72) Inventor: Fournier Albert, 80090 Amiens (FR); Achard Jean-Michel, 87400 Saint Martin Terressus (FR); Fernandez Leonardo A., Connecticut 06437 Guilford (US)
(74) Representative: Callon de Lamarck, Jean-Robert

(57) **Abstract**

Use of angiotensin II AT1-receptor blocker (ARB), for the preparation of a drug for primary and secondary stroke prevention, specifically in patients with low prevalence or absence of coronary heart disease (CHD).

## Description

### TECHNICAL FIELD.

The present invention relates to pharmaceutical products for the treatment of hypertension. More particularly, the present invention concerns the administration of AT1-receptor blockers (ARB) for brain antiischemia protection of patients with low prevalence or absence of CHD, and the administration of ARB and angiotensin II for very early treatment of acute stroke.

### BACKGROUND OF THE INVENTION.

### Primary and secondary prevention of stroke.

Epidemiological data have shown that in the USA, age-adjusted stroke incidence decline has dramatically slowed down and may actually be increasing. Furthermore stroke death rate annual decrease has been reversed since 1980 in spite of better control of blood pressure, dyslipidemia and smoking as well as a better treatment of acute stroke as evidenced by decreased case-fatality.

The main potential factors favoring these ominous trends are the increasing prevalence of obesity and diabetes, and paradoxically a better treatment and prevention of CHD and heart failure than of stroke because it results in an increasing population at higher risk of stroke since these cardiac complications are per se stroke risk factors. This may have been favored by the prescription decrease of antihypertensive drugs stimulating angiotensin II-formation in favor of drugs suppressing this formation between the seventies and nineties since they are less protective against stroke than against CHD. Therefore a dramatic increase of stroke burden is expected in the next decades.

Angiotensin II (All) is a potent vasoconstrictor. Its generation results from the enzymatic action of renin on a blood plasma alpha.2-globulin, angiotensinogen which produces angiotensin I (AI). AI is then converted by angiotensin converting enzyme (ACE) to the octapeptide hormone AII. All has been implicated as mediator of hypertension through its AT1 receptor. Therefore, antihypertensive agents called ACE inhibitiors (ACEI), which decrease the production of All via angiotensin converting enzyme, and ATI-receptor antagonists, which inhibit the activation of AT1-receptor, no matter the pathway of All biosynthesis, are often used in the treatment of hypertension.

ACE inhibitors (e.g. captopril, enalapril or lisinopril) besides inhibiting the conversion of angiotensin I to angiotensin II also potentiates the effects of systemic bradykinin since they inhibit its catabolism, whereas AT1-selective AII receptor antagonists (e.g. losartan) selectively inhibit the function of All at the AT1 receptor site, but activate AT2-receptors which in case of ischemia insult are overexpressed and locally induce a well targeted vasodilatation related also to bradykinin overproduction.

In certain clinical trials, namely in patients whith coronary heart disease (CHD), ACEI may induce a marked blood pressure-independent reduction of both cardiac complications and stroke, by blunting the vasoconstrictive and proatherothrombotic effects mediated by the AT 1 -receptors.

However, in patients with low CHD prevalence, ACEI for example captopril and perindopril have been reported to increase the blood pressure-independent risk of stroke. Thus the treatment of hypertension with ACEI is not optimal in these patients.

In rodent models of acute brain ischemia, it has been shown that the losartan ARB protects better than the ACEI enalapril against ischemic events, specially the cerebral ones, by favouring the rapid recruitment of collateral circulation. Losartan potassium (losartan) has been disclosed in a U.S. Pat. No.5,138,069. It has been demonstrated in case of abrupt ligation of the carotid in the gerbil that previous administration of losartan protects against fatal stroke whereas previous co-administration of enalapril abolishes this protection. The inventors suggest the hypothesis that ATI-receptors antagonists (AT1RA or ARB) by blocking only AT1-receptors but not non-AT1-receptors (AT2 and

AT4) would stimulate non- AT1-receptors since they increase the production of All secondarily to the inhibition of the negative feedback of All on renin secretion. Whether these differentials effects of losartan and enelapril are drug or drug-class related has not yet been demonstrated. These differences need yet to be demonstrated with other ACEI and ARB.

### Treatment of acute stroke.

In patients with acute stroke, early treatment of hypertension (even with calcium antagonist with experimentally proven neuroprotective effects), has given either neutral or even deleterious effects when DBP decreased below 10% of its baseline. Considering the greater protective effect of losartan, comparatively to enalapril at isohypotensive dose in the gerbil model of acute stroke, the inventors suggest that ARB induce specific antiischemic effect in case of stroke, specially through activation of the AT2 and/or AT4 receptors which are specifically overexpressed in the ischemia brain. This activation may be furthermore enhanced by rapid intravenous infusion of All. Indeed in the gerbil model of stroke by rapid carotid ligation this infusion, decreased mortality and increased ipsilateral cerebral blood flow.

### SUMMARY OF THE INVENTION

The first aspect of the invention is the definition of the preferential indication of ARB over ACEI for the prevention of stroke in patients with a higher risk of stroke than of cardiac complication because of low prevalence or absence of coronary heart disease (CHD).The rationale for this choice is namely the critical review of the trials by the inventors which pointed that these patients are hypertensive elderly patients without CHD, or patients with a history of stroke, whatever their BP, but without CHD. These populations are those in which an inverse link is observed between stroke risk and stimulation of of AII formation by antihypertensive drugs.

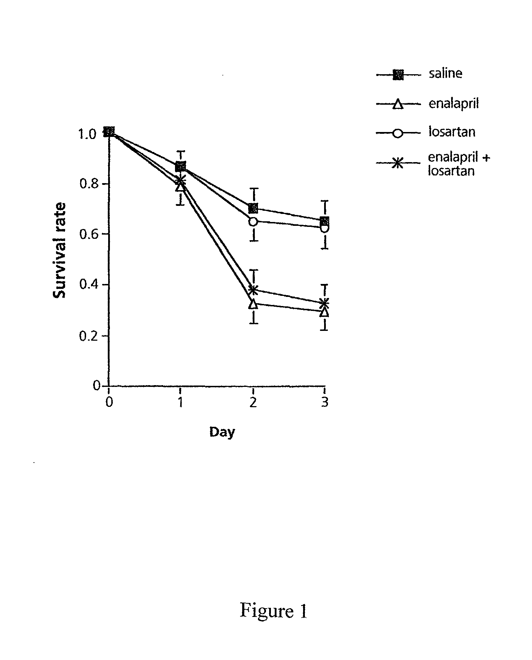

The inventors have shown in rodent models of acute brain ischemia that the ARB might be more protective than ACEI: ARBs trigger stroke-protective mechanisms, through stimulation of angiotensin non-AT1-receptors. ARB increase All-synthesis by blunting the AT1-mediated inhibition of renin secretion, and they improve the survival of the gerbil when administered before carotid ligation comparatively to ACEI administration (figure 1). These mechanisms may be hemodynamic (activation of collateral circulation recruitment by bradykinin-mediated vasodilatation) or metabolic (increased neuronal resistance to anoxia). The efficiency of these mechanisms is further enhanced by ischemia-induced overexpression of AT2-receptors. This may explain the greater gerbil survival after carotid ligation with ARB than with ACEI preadministration through a more appropriate targeting of bradykinin-mediated vasodilation only to ischemic zone, and not to non ischemic tissue (steal phenomenon) as it may occur with the systemic vasodilation linked to the increase of systemic levels of bradykinin induced by ACEI. The inventors show that the preponderence of the protective effect of A1l on the BP-independent stroke risk (by activation of non-AT1-receptor mediated brain anti-ischemic mechanisms) over the deleterious effect of All on this risk (by activating ATI- receptor mediated proatheroinflammatory effects leading to plaque destabilization, thrombosis or emboli of coronary and cerebral arteries), is inversely related to the dependence of stroke risk upon the risk of cardiac complications which is related to the initial prevalence of coronary heart disease (CHD).

Thus the first aspect of the invention is the specific use of angiotensin II AT1-receptor blocker (ARB), for the preparation of a drug for primary and secondary stroke prevention, specifically in patients with low prevalence or absence of coronary heart disease (CHD). This use of ARB takes advantage of the BP-independent (blood pressure independent) brain antiischemic effect of ARB which occur through non-AT1-receptors activation. The term low prevalence refers to a CHD level less than 25%, preferably less than 16%.

The ARB agent is typically chosen in the group consisting of candesartan, eprosartan, irbesartan, losartan, telmisartan, tasosartan, valsartan, olmesartan.

For stroke prevention, the ARB is administered on a chronic basis typically at a dose ranging between 8 mg and 300 mg for an undefinite duration, for instance 50 to 100 mg for losartan, 75 to 300 mg for irbesartan, 8 to 32 mg for candesartan.

In a preferred embodiment, the ARB is coadministrated with low dose thiazides equivalent to 25-50 mg of hydrochlorothiazide. The thiazide could be hydrochlorothiazide, chlorthalidone, indapamide (2,5 mg). Thiazides make a drug class which has been shown to be both cardiac and cerebral-protective in uncomplicated hypertension trials against placebo as well as in stroke secondary prevention trials. Furthermore they synergistically act with both ACEI and ARB in blood pressure lowering.

The second aspect of the invention is related to the treatment of patients with acute stroke. In contrast to the current state of the art, the inventors give strong support to the advantageous co-administration of AII and ARB for very early treatment of acute stroke. Indeed, the inventors give strong support to the hypothesis that the superiority of ARB over ACEI in decreasing the severity of acute stroke in the gerbil as well as the beneficial effect of early intravenous infusion of angiotensin II in this model, can be combined in the treatment of patients with acute stroke in order to maintain an optimal blood pressure level while maximally activating the antiischemic non-ATI-receptor mediated mechanisms .

Thus the invention relates also to the use of ARB, in combination with angiotensin II, for the preparation of a drug against ischemic accident administrated as soon as possible after stroke onset for duration of 3 to 7 days. The administration of ARB and angiotensin II may be simultaneous, separate, or sequential.

The sequence of administration of the two therapeutical elements will depend on the level of blood pressure observed in the patients, a pressure of DBP between 85-104 during the first 3 days being considered as optimal.
- When the DBP is in this optimal range the dose of ARB and AII may be the following :
   - first 30 hours : ARB half usual dose, All infusion 1-2,5 ng/kg/min
   - 30-72 hours : ARB usual dose, All 2,5 ng/kg/min
   - days 4-5 : ARB usual dose, AII discontinuation
- When the DBP is above this optimal range only ARB are used at doses which be increased successively from the usual dose to 3 times the usual dose. However the
   BP lowering should be not less than 10% of the basal value in the first three days.
- When the initial DBP is below 85 mmHg only All infusion will be used at progressively increasing dose from 0.5 to 20 ng/kg/min.

The invention also relates to:
- a method of prevention of primary and secondary stroke comprising administration of ARB specifically in patients with low CHD;
- a method of treatment of acute stroke comprising administration of ARB and angiotensin II as soon as possible after stroke onset.

The invention further relates to pharmaceutical compositions (typically kit preparations) comprising at least an ARB compound and angiotensin II.

The invention further relates to the use of selective agonists of non-AT1-receptors (typically angiotensin II-AT2-receptors and angiotensin IV-AT4-receptors), i.e either natural angiotensin II or angiotensin IV or organic compounds mimicking the action of these hormones, for the preparation of a drug against acute stroke.

The invention further relates to pharmaceutical preparations comprising at least an ARB for parenteral administration (intravenous, intracerebroventrivular, intraarterial). The pharmaceutical preparations may be formulated also for oral administration in solid or liquid form.

Moreover, a combination of one or more of the ARB agents from the group listed above may be combined with a diuretic or a calcium antagonist well known to have BP-independent stroke protective effect.

The pharmaceutical compositions of this invention can be administered either systemically or locally to humans and other animals. Systemic routes include oral, parenteral, intracisternal, intraperitoneal, trans-cutaneous (by injection or patch), buccal, sub-lingual administration, or by means of an oral or nasal spray. The term "parenteral" administration as used herein refers to modes of administration which include intravenous, intramuscular, intraperitoneal, subcutaneous and intraarterial injection and infusion. Local administration routes include intracerebroventricular injection.

Pharmaceutical compositions of this invention for parenteral injection comprise typically pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carrier, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.These compositions may also contain adjuvants such as preservatives, wetting agents, emulsifying agents, and dispersing agents. Prevention of the action of microorganisms may be ensured by the inclusion of various and bacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminum monostearate and gelatin.

In some cases, in order to prolong the effect of the drug it is desirable to slow the release or absorption of the drug following subcutaneous or intramuscular rejection. This may be accomplished by the use of a liquid suspension of crystalline or amorphous material with low water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(othoesters) and poly(anhydrides). Depot injectable formulations are also prepared by entrapping the drug in liposomes or microemulsions which are compatible with body tissues. The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and I) lubricants such as talc, calcium stearate, magnesium stearate, solid polyethylene glycols, sodium lauryl sulfate, and mixtures thereof. In the case, of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, ground nut corn, germ olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic. Methods for the formation of liposomes are known in the art. See, for example, Prescott, Ed., Methods in Cell Biology, Volume XIV Academic Press, New York, N.Y. (1976), p. 33 et seq.

Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is well known within the medical art to determine the proper dose for a particular patient by the "dose titration" method. In this method, the patient is started with a dose of the drug compound at a level lower than that required to achieve the desired therapeutic effect. The dose is then gradually increased until the desired effect is achieved. Starting dosage levels for an already commercially available therapeutic agent of the classes discussed above can be derived from the information already available on the dosages employed for the use of the compound as an antihypertensive agent.

For the preferred therapeutic agents in the use of the present invention for brain antiischemic protection, generally dosage levels of about 4 mg to about 300 mg for a mean body weight of 50-80 kg, the dose being appropriately adjusted according body weight outside this range.

For the use for treatment of acute stroke, the ARB is an ARB whit short duration of action. Besides angiotensin II, angiotensin IV, may be used as well as organic compounds with selective property on AT2 and AT4 receptors.

The pharmaceutically acceptable salts of the present invention refer to common salts. Examples of such salts include acid addition salts with mineral acids such as hydrochloric acid, hydrobromic acid, hydroiodic acid, sulfuric acid, nitric acid and phosphoric acid, acid addition salts with organic acids such as formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid and aspartic acid, salts with organic amines such as lysine, and salts with inorganic bases such as a sodium salt, a potassium salt and a calcium salt. In addition, the pharmaceutically acceptable salts of the present invention also include hydrates thereof and solvates thereof with pharmaceutically acceptable solvents such as ethanol.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easton, Pa., 18th Edition (1990). Pharmaceutical compositions according to this invention may contain 0.1%-95% of a compound of this invention, preferably 1%-70%. In any event, the composition or formulation to be administered will contain a quantity of a compound according to this invention in an amount effective to treat the disease/condition of the subject being treated, e.g., congestive ischemic accident. For the brain protection, thiazide, indapamide, calcium antagonists and ARB or their pharmaceutically acceptable salts in a pharmaceutically acceptable carrier, may be administrated with either concomitant therapy or a fixed dose combination. Concomitant therapy with thiazide and ARB would include the sequential administration of members from the two classes of compounds. A fixed dose combination would be in the form of a tablet or capsule and comprises between 12.5 mg to about 50 mg of thiazide or indapamide at 1,5 - 2,5 mg. As regards the dosage of ARB it depends on the molecule chosen as described above. A preferred embodiment is for exemple a pharmaceutical composition consisting of about 12,5 mg thiazide maleate and 50 mg losartan potassium and a pharmaceutical carrier.

The ARB compounds and angiotensin II or IV may be administered separately and prepared in a kit form. The kit includes container means for containing the separate compositions such as a divided bottle or a divided foil packet, however, the separate compositions may also be contained within a single, undivided container. Typically the kit includes directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

Further objects and advantages of the invention will be described in the following description.

### BRIEF DESCRIPTION OF THE DRAWINGS FIGURES IN THE DRAWING:

Figure 1 describes survival after right carotid ligation. Oral pretreatment 30 minutes before surgery: saline 0.5 ml; n= 37; enalapril, 10 mg/kg; n=37; losartan 50 mg/kg; n=37; losartan + enalapril, same doses; n=37.
   Kaplan-Meier survival analysis, log-rank (Mantel-Cox) test:
   - saline vs. losartan, ns
   - saline vs. enalapril, p=0.004
   - losartan vs. enalapril, p=0.008
   - enalapril vs. losartan + enalapril, ns.
Figure 2 describes survival rate after right carotid ligation. Intravenous pretreatment with saline (n=53), 3 mg/kg lisinopril (n=44) or 1 mg/kg candesartan (n=44).
   Kaplan-Meier survival analysis, log-rank (Mantel-Cox) test:
   - saline vs. candesartan, ns
   - saline vs. lisinopril, p=0.0004
   - candesartan vs. lisinopril, p<0.0001.

### DETAILED DESCRIPTION

### METHODS

Adult male gerbils (Meriones unguiculatus) weighing 60-75 g, were purchased from Janvier (Le Genest St Isle 53940, France) and kept in a controlled-temperature (24°C). They were fed rat chow and tape water was given *ad libitum.*

In a first set of experiments, gerbils were pretreated 2 hours prior to surgery with an oral bolus of either losartan (50 mg/kg), enalapril (10 mg/kg) or the combinaison of the two, dissolved in 0.5 ml saline, or saline alone by gastric gavage. Surgical procedures were performed under sodium penthobarbital anaesthesia (60 mg/kg of body weight intraperitoneally). After making a midline neck incision, the common carotid artery was exposed and then ligated with a silk thread. The skin was sutured and the animals returned to their cages and allowed to recover from anaesthesia. In a subset of animals similarly pretreated the abdominal aorta was exposed and canulated for invasive measurement of blood pressure (BP) via an external transducer. BP was measured after 5 minutes, three times at 1 minute intervals, and the canula rinsed with 0.1 ml saline solution after each measurement.

In a second set of experiments, gerbils were anaesthetized and the penian vein was exposed and canulated. Candesartan cilexetil (1 mg/kg), lisinopril (3 mg/kg), or the vehicle, saline (0.5ml) was administered intravenously. Unilateral carotid ligation was performed 20-30 minutes following pretreatment. A subset of animals was similarly treated for subsequent invasive measurements of BP.

Results are reported as mean ± standard error of mean (SEM). Mortality rate in each group of animals was assessed at 24, 48 and 72 hours and a log-rank test was used for statistical analysis of the Kaplan-Meier survival software (Abacus concept, Inc, Berkeley, CA 1996). Student t-test and ANOVA were used for comparison of BP levels.

### RESULTS

### Effect of losartan and enalapril

Survival rates following carotid ligation in losartan- and enalapril-pretreated gerbils are shown in Figure 1. Survival rates at day three in the control group (treated with saline) was 64.9 ±7.8%, (n=37) and 62.2 ±8% in the losartan group (n=37); survival was half that in the two groups pretreated with enalapril, either alone (29.717.5%, n=37) or in combination with losartan (32.4±7.7%, n=37). Statistical analysis of the Kaplan-Meier survival curves showed that the mortality rate of losartan-pretreated animals was not statistically different from that of the controls, but their mortality was significantly increased by enalapril (p=0.004 vs. saline and 0.008 vs. losartan) and by the combination of enalapril and losartan (p= 0.008 vs. saline and 0.02 vs. losartan). The mean BP in saline-pretreated anaesthetized animals was 85.4±8mmHg, (n=5), and was significantly lowered by pretreatment with enalapril (70.0±5.6 mmHg, n=5; p=0.003) or losartan (68.0±4.7 mmHg, n=5; p=0.004), but there was no difference between the losartan and enalapril groups. Thus, the deleterious effect of enalapril on survival rate was independent of BP-lowering.

### Effect of candesartan and lisinopril

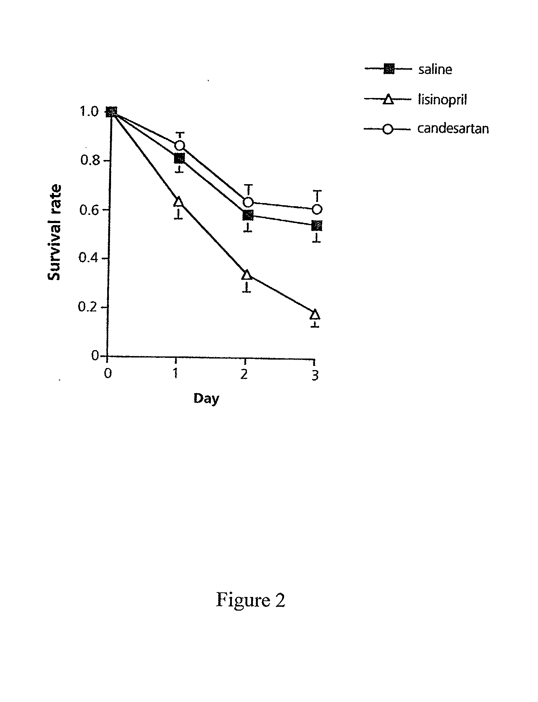

To further examine whether these findings were the result of intrinsic drug class differences between ACE-I and AT-receptor antagonists, or alternatively result from some specific property of losartan or enalapril unrelated to their action on the RAS, the effect of candesartan was compared with that of lisinopril in another set of experiments prior to carotid ligation (Figure 2)

The survival rate at day three was 57.7±6.8% in the control group (n=53) and 61.4±7.3% in the candesartan group (n=44); survival was again much lower (18.2±5.8%) in the group pretreated with the ACE-I, lisinopril (n=44). Statistical analysis of the Kaplan-Meier survival curve showed that the mortality rate of candesartan-pretreated animals did not differ from that of the controls but that mortality was significantly increased by lisinopril (p=0.0004 vs. saline, and <0.0001 vs. candesartan). There was no difference in intra-aortic systolic and diastolic BP (mmHg) between the lisinopril and candesartan groups (lisinopril (n=3): 89 ±1/66±2; candesartan (n=3): 86±1.7/62±2), but BP was significantly lower than in saline-pretreated gerbils (controls (n=5):122±16/96±14).

## Claims

1. Use of angiotensin II AT1-receptor blocker (ARB), for the preparation of a drug for primary and secondary stroke prevention, specifically in patients with low prevalence or absence of coronary heart disease (CHD).

2. Use of angiotensin II ATI-receptor blocker (ARB) and thiazide, for the preparation of a drug for primary and secondary stroke prevention, specifically in patients with low prevalence or absence of coronary heart disease (CHD).

3. Use of claim 1 or 2 wherein said ARB is administered on a chronic basis.

4. Use of ATI receptor blocker (ARB), in combination with angiotensin II, for the preparation of a drug against acute stroke, the drug being administrated as soon as possible after stroke onset, typically for 3 to 7 days.

5. Use according to claim 4 comprising simultaneous, separate, or sequential administration of ARB and angiotensin II.

6. Use of claim 1 to 5 wherein said ARB agent is chosen in the group consisting of valsartan, eprosartan, irbesartan, losartan, candesartan, olmesartan.

7. Pharmaceutical preparation, typically in a kit form, comprising at least an ARB and at least angiotensin II.

8. Use of selective agonists of angiotensin II-AT2-receptors and of angiotensin IV-AT4-receptors, i.e either natural angiotensin II or angiotensin IV or organic compounds mimicking the action of these hormones, for the preparation of a drug against acute stroke.
